# EUROPÄISCHE PATENTANMELDUNG

(11) **EP 4 461 258 A1**
(43) Veröffentlichungstag der Anmeldung: **13.11.2024**
(21) Anmeldenummer: 23173077.1
(22) Anmeldetag: 12.05.2023
(51) Int. Cl.: A61C 17/08, A61C 5/90

(54) **ABDECKVORRICHTUNG ZUM ABDECKEN DER LIPPEN UND MUNDWINKEL**

(71) Anmelder: Ivoclar Vivadent AG, 9494 Schaan (LI)
(72) Erfinder: Lichtensteiger, Markus, 9462 Montlingen (CH)
(74) Vertreter: Baldus, Oliver

(57) **Zusammenfassung**

Die vorliegende Erfindung betrifft eine Abdeckvorrichtung (100) zum Abdecken der Lippen und Mundwinkel eines Patienten während einer Behandlung, die einen Kunststoff mit Duftmolekülen umfasst.

## Beschreibung

Die vorliegende Erfindung betrifft eine Abdeckvorrichtung zum Abdecken der Lippen und Mundwinkel eines Patienten während einer Behandlung und Verfahren zum Herstellen einer Abdeckvorrichtung.

Es ist die technische Aufgabe der vorliegenden Erfindung, eine dentale Abdeckvorrichtung oder einen Speichelsauger bereitzustellen, die einen angenehmeren Komfort bieten.

Diese Aufgabe wird durch Gegenstände nach den unabhängigen Ansprüchen gelöst. Technisch vorteilhafte Ausführungsformen sind Gegenstand der abhängigen Ansprüche, der Beschreibung und der Zeichnungen.

Gemäß einem ersten Aspekt wird die technische Aufgabe durch eine Abdeckvorrichtung zum Abdecken der Lippen und Mundwinkel eines Patienten während einer Behandlung gelöst, die einen Kunststoff mit Duftmolekülen umfasst. Durch die Abdeckvorrichtung wird der technische Vorteil erreicht, dass sich der Tragekomfort der Abdeckvorrichtung erhöht.

In einer technisch vorteilhaften Ausführungsform der Abdeckvorrichtung ist der Kunststoff aus Polypropylen oder Polyethylen gebildet. Dadurch wird beispielsweise der technische Vorteil erreicht, dass die Duftmoleküle in besonders geeigneten Kunststoffen eingebettet werden können und nach und nach abgegeben werden.

In einer weiteren technisch vorteilhaften Ausführungsform der Abdeckvorrichtung umfasst die Abdeckvorrichtung ein elastisches Lippen-Spannelement, ein elastisches Vestibulär-Spannelement und einen die Spannelemente verbindenden Folienabschnitt. Dadurch wird beispielsweise der technische Vorteil erreicht, dass das Abdeckelement effizient in der Mundöffnung eines Patienten eingesetzt werden kann.

In einer weiteren technisch vorteilhaften Ausführungsform der Abdeckvorrichtung ist der Kunststoff mit den Duftmolekülen in dem Lippen-Spannelement und/oder dem Vestibulär-Spannelement angeordnet. Dadurch wird beispielsweise der technische Vorteil erreicht, dass die Duftmoleküle an besonders geeigneten Stellen ausströmen.

In einer weiteren technisch vorteilhaften Ausführungsform der Abdeckvorrichtung ist der Kunststoff mit den Duftmolekülen lediglich in einem Teilbereich des Lippen-Spannelements und/oder des Vestibulär-Spannelements angeordnet. Dadurch wird beispielsweise der technische Vorteil erreicht, dass ein Materialaufwand für den Kunststoff mit den Duftmolekülen verringert wird.

In einer weiteren technisch vorteilhaften Ausführungsform der Abdeckvorrichtung ist der Teilbereich optisch erkennbar oder optisch hervorgehoben. Dadurch wird beispielsweise der technische Vorteil erreicht, dass der Teilbereich beim Einsetzen der Abdeckvorrichtung direkt unter einer Nase des Patienten angeordnet werden kann.

In einer weiteren technisch vorteilhaften Ausführungsform der Abdeckvorrichtung ist der Kunststoff mit den Duftmolekülen in dem Folienabschnitt angeordnet. Dadurch wird beispielsweise der technische Vorteil erreicht, dass eine große Duftfläche erreicht wird und lediglich eine geringe Menge des Kunststoffs mit Duftmolekülen verwendet zu werden braucht.

In einer weiteren technisch vorteilhaften Ausführungsform der Abdeckvorrichtung ist der Kunststoff mit den Duftmolekülen an einer Außenseite der Abdeckvorrichtung angeordnet. Dadurch wird beispielsweise der technische Vorteil erreicht, dass ein Blockieren des Ausströmens der Duftmoleküle durch die Haut verhindert wird.

In einer weiteren technisch vorteilhaften Ausführungsform der Abdeckvorrichtung ist der Kunststoff mit den Duftmolekülen lediglich in einem Teilbereich der Abdeckvorrichtung angeordnet. Dadurch wird beispielsweise der technische Vorteil erreicht, dass der Kunststoff mit den Duftmolekülen an gezielten Stellen eingesetzt werden kann.

In einer weiteren technisch vorteilhaften Ausführungsform der Abdeckvorrichtung beträgt der Gewichtsanteil des Kunststoffs mit den Duftmolekülen zwischen 0,5% und 5%. Dadurch wird beispielsweise der technische Vorteil erreicht, dass Kunststoff mit Duftmolekülen besonders wirksam verwendet wird.

Gemäß einem zweiten Aspekt wird die Aufgabe durch ein Verfahren zum Herstellen einer Abdeckvorrichtung zum Abdecken der Lippen und Mundwinkel eines Patienten während einer Behandlung gelöst, bei dem ein Kunststoff mit Duftmolekülen in eine Spritzgussform eingespritzt wird. Dadurch werden die gleichen technischen Vorteile wie durch die Abdeckvorrichtung nach dem ersten Aspekt erreicht.

Gemäß einem dritten Aspekt wird die Aufgabe durch einen Speichelsauger zum Absaugen von Speichel aus dem Mund eines Patienten gelöst, der einen Kunststoff mit Duftmolekülen umfasst. Durch den Speichelsauger wird ebenfalls der technische Vorteil erreicht, dass der Komfort bei einer Verwendung des Speichelsaugers erhöht.

In einer technisch vorteilhaften Ausführungsform des Speichelsaugers umfasst der Speichelsauger ein Röhrchen, in dem der Kunststoff mit den Duftmolekülen angeordnet ist. Dadurch wird beispielsweise der technische Vorteil erreicht, dass der Kunststoff mit den Duftmolekülen besonders wirksam eingesetzt wird.

In einer technisch vorteilhaften Ausführungsform des Speichelsaugers ist der Kunststoff mit den Duftmolekülen an der Spitze oder dem Ende des Röhrchens angeordnet. Dadurch wird beispielsweise der technische Vorteil erreicht, dass der Kunststoff mit den Duftmolekülen nur in Teilbereichen eingesetzt wird und ein Materialaufwand sinkt.

Gemäß einem vierten Aspekt wird die Aufgabe durch ein Verfahren zum Herstellen eines Speichelsaugers gelöst, bei dem ein Kunststoff mit Duftmolekülen in eine Spritzgussform eingespritzt wird. Dadurch werden die gleichen technischen Vorteile wie durch den Speichelsauger nach dem dritten Aspekt erreicht.

Ausführungsbeispiele der Erfindung sind in den Zeichnungen dargestellt und werden im Folgenden näher beschrieben.

Es zeigen:
- Fig. 1: eine Ansicht einer Abdeckvorrichtung zum Abdecken der Lippen und Mundwinkel eines Patienten während einer Behandlung; und
- Fig. 2: eine Ansicht eines Speichelsaugers zum Absaugen von Speichel aus dem Mund eines Patienten.

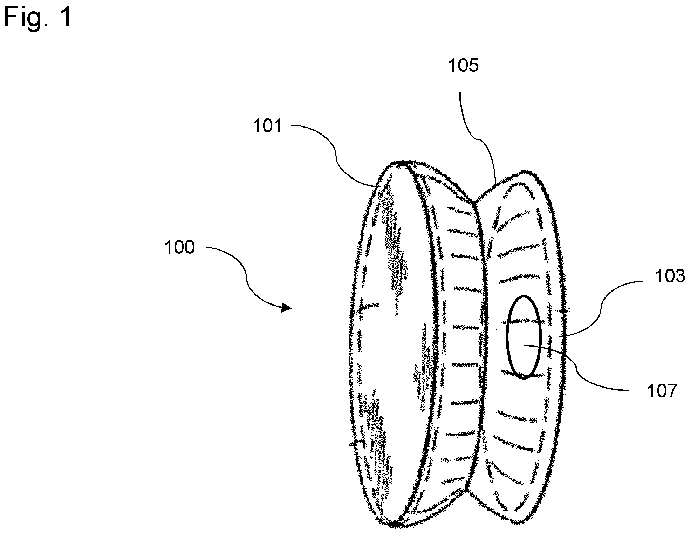

Fig. 1 zeigt eine Ansicht einer Abdeckvorrichtung 100 zum Abdecken der Lippen und Mundwinkel eines Patienten während einer Behandlung, wie beispielsweise einen Mund-Wangenhalter (Optragate). Dadurch wird ein hygienischer Zugang zum Mund eines Patienten während einer Behandlung ermöglicht. Die Lippen und Mundwinkel sind mit der Abdeckvorrichtung 100 bedeckt.

Die Abdeckvorrichtung 100 umfasst ein elastisches Lippen-Spannelement 101, ein elastisches Vestibulär-Spannelement 103 und einen die Spannelemente 101 und 103 verbindenden Folienabschnitt 105. Das Lippen-Spannelement 101 ist als Spannring gebildet und spannt sich außerhalb des Mundes auf. Das Vestibulär-Spannelement 103 ist ebenfalls als Spannring gebildet und spannt sich innerhalb des Mundes auf. Das Lippen-Spannelement 101 und/oder das Vestibulär-Spannelement 103 können kreisrund oder oval ausgebildet sein.

Der Folienabschnitt 105 zwischen dem Lippen-Spannelement 101 und dem Vestibulär-Spannelement 103 bildet ein Abdeckmittel und legt sich dabei um den Rand der Mundöffnung. Dadurch kann ein steriler Zugang zur Mundöffnung erreicht werden. Die Abdeckvorrichtung 100, d.h. das Lippen-Spannelement 101, das Vestibulär-Spannelement 103 und/oder der Folienabschnitt 105, kann aus einem Kunststoff, wie beispielsweise Polypropylen (PP) oder Polyethylen (PE), mittels eines Spritzgussverfahrens hergestellt werden. Die Abdeckvorrichtung 100 bietet eine einzigartige Flexibilität und Elastizität und einen angenehmen Tragekomfort. Zudem erleichtert die Abdeckvorrichtung 100 dem Patienten das Offenhalten des Mundes. Die Abdeckvorrichtung 100 ermöglicht ein gleichmäßiges Abhalten von Lippen und Wangen, ein frei zugängliches, übersichtliches Behandlungsfeld und eine effektive und schnelle relative Trockenlegung.

Zusätzlich umfasst die Abdeckvorrichtung 100 einen Kunststoff mit Duftmolekülen (Duft-Masterbatch). Diese können beispielsweise hergestellt werden, indem der Kunststoff mit einem Duftöl versetzt wird. Der Kunststoff kann beispielsweise ein Polypropylen oder Polyethylen sein. Im Dentalbereich kann beispielsweise ein Pfefferminzduft für die Duftmoleküle verwendet werden. Es können aber auch andere Duftmoleküle verwendet werden, wie beispielsweise für einen Zitrusduft, einen Orangenduft, einen Blumenduft, einen Anisduft oder einen Fichtennadelduft. Im Allgemeinen kann auch jedes andere Duftmolekül verwendet werden.

Der Gewichtsanteil des Kunststoffs mit Duftmolekülen beträgt beispielsweise zwischen 0,5% und 5%. Es können je nach Verwendung und Duftintensität auch mehr als 5% verwendet werden. Die Duftmoleküle können durch Migration des Duftstoffes an die Oberfläche der Abdeckvorrichtung 100 treten.

Der Kunststoff mit den Duftmolekülen kann mit dem übrigen Kunststoff vermischt sein, aus dem die Abdeckvorrichtung 100 gebildet ist. In diesem Fall sind die Duftmoleküle in der gesamten Abdeckvorrichtung 100 gleichmäßig verteilt. Der Kunststoff mit den Duftmolekülen kann jedoch auch lediglich in einem Teilbereich 107 des Lippen-Spannelements 101 und/oder des Vestibulär-Spannelements 103 oder in dem Folienabschnitt 105 angeordnet sein. In diesem Fall ist nur ein Teilbereich der Abdeckvorrichtung 100 mit den Duftmolekülen versehen. Die übrige Abdeckvorrichtung 100 ist frei von Duftmolekülen.

Zudem kann der Teilbereich, in dem die Duftmoleküle angeordnet sind, farblich hervorgehoben oder in anderer Weise markiert sein. In diesem Fall ist ein Benutzer in der Lage, die Abdeckvorrichtung 100 so einzusetzen, dass der Teilbereich mit den Duftmolekülen direkt unter einer Nase angeordnet wird. Sind die Duftmoleküle jedoch gleichmäßig in der Abdeckvorrichtung 100 verteilt, wird der Vorteil erreicht, dass die Abdeckvorrichtung 100 in einer beliebigen Position eingesetzt werden kann.

Die Duftmoleküle können auch lediglich an einer Außenseite der Abdeckvorrichtung 100 angeordnet sein, d.h. an einer der Haut des Patienten abgewandten Seite. An dieser Seite können die Duftmoleküle effizient austreten und in die Umgebung gelangen.

Bei einem Verfahren zum Herstellen der Abdeckvorrichtung 100 wird beispielsweise im ersten Schritt ein Kunststoff mit Duftmolekülen mittels eines Spritzgussverfahrens in eine Spritzgussform eingespritzt. Daneben können auch noch andere Kunststoffe in die Spritzgussform eingespritzt werden. In einem zweiten Schritt wird anschließend die Abdeckvorrichtung 100 aus der Spritzgussform entnommen.

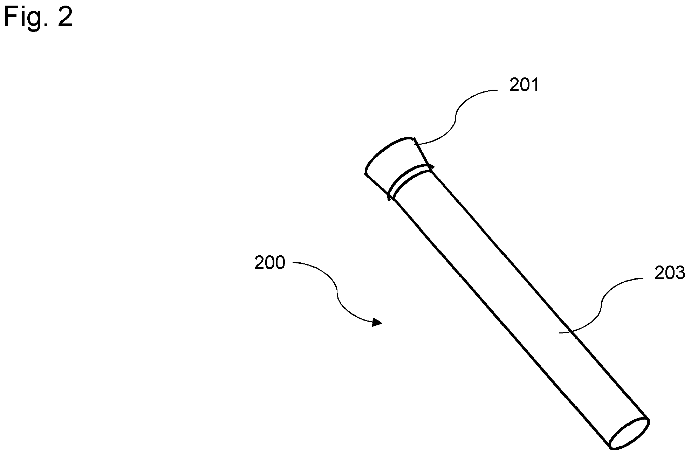

Fig. 2 zeigt eine Ansicht eines Speichelsaugers 200 zum Absaugen von Speichel aus dem Mund eines Patienten. Der Speichelsauger 200 ist durch ein biegsames Röhrchen 203 aus Kunststoff wie beispielsweise Polypropylen (PP) oder Polyethylen (PE) gebildet. Dieses wird über ein Anschlussstück 201 und einen flexiblen Schlauch mit einer Saugvorrichtung verbunden.

Der Speichelsauger 200 umfasst ebenfalls einen Kunststoff mit Duftmolekülen. Die Duftmoleküle sind in dem Röhrchen 203 angeordnet. Diese können nur in Teilbereichen vorgesehen sein, wie beispielsweise der Spitze oder dem Ende des Röhrchens 203 oder gleichmäßig in dem Röhrchen 203 verteilt sein. Der Gewichtsanteil des Kunststoffs mit Duftmolekülen beträgt beispielsweise zwischen 0,5% und 5%. Es können je nach Verwendung und Duftintensität auch mehr als 5% verwendet werden.

Bei einem Verfahren zum Herstellen des Speichelsaugers wird im ersten Schritt ein Kunststoff mit Duftmolekülen mittels eines Spritzgussverfahrens in eine Spritzgussform eingespritzt. Daneben können auch noch andere Kunststoffe in die Spritzgussform eingespritzt werden. In einem zweiten Schritt wird anschließend der Speichelsauger aus der Spritzgussform entnommen.

Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein.

Der Schutzbereich der vorliegenden Erfindung ist durch die Ansprüche gegeben und wird durch die in der Beschreibung erläuterten oder den Figuren gezeigten Merkmale nicht beschränkt.

### BEZUGSZEICHENLISTE

- 100: Abdeckvorrichtung
- 101: Lippen-Spannelement
- 103: Vestibulär-Spannelement
- 105: Folienabschnitt
- 107: Teilbereich

- 200: Speichelsauger
- 201: Anschlussstück
- 203: Röhrchen

## Patentansprüche

1. Abdeckvorrichtung (100) zum Abdecken der Lippen und Mundwinkel eines Patienten während einer Behandlung, die einen Kunststoff mit Duftmolekülen umfasst.

2. Abdeckvorrichtung (100) nach Anspruch 1, wobei der Kunststoff aus Polypropylen oder Polyethylen gebildet ist.

3. Abdeckvorrichtung (100) nach einem der vorangehenden Ansprüche, wobei die Abdeckvorrichtung (100) ein elastisches Lippen-Spannelement (101), ein elastisches Vestibulär-Spannelement (103) und einen die Spannelemente verbindenden Folienabschnitt (105) umfasst.

4. Abdeckvorrichtung (100) nach Anspruch 3, wobei der Kunststoff mit den Duftmolekülen in dem Lippen-Spannelement (101) und/oder dem Vestibulär-Spannelement (103) angeordnet ist.

5. Abdeckvorrichtung (100) nach Anspruch 4, wobei der Kunststoff mit den Duftmolekülen lediglich in einem Teilbereich (107) des Lippen-Spannelements (101) und/oder des Vestibulär-Spannelements (103) angeordnet ist.

6. Abdeckvorrichtung (100) nach Anspruch 5, wobei der Teilbereich (107) optisch erkennbar oder optisch hervorgehoben ist.

7. Abdeckvorrichtung (100) nach einem der Ansprüche 3 bis 6, wobei der Kunststoff mit den Duftmolekülen in dem Folienabschnitt (105) angeordnet ist.

8. Abdeckvorrichtung (100) nach einem der vorangehenden Ansprüche, wobei der Kunststoff mit den Duftmolekülen an einer Außenseite der Abdeckvorrichtung (100) angeordnet ist.

9. Abdeckvorrichtung (100) nach einem der vorangehenden Ansprüche, wobei der Kunststoff mit den Duftmolekülen lediglich in einem Teilbereich der Abdeckvorrichtung (100) angeordnet

10. Abdeckvorrichtung (100) nach einem der vorangehenden Ansprüche, wobei der Gewichtsanteil des Kunststoffs mit den Duftmolekülen zwischen 0,5% und 5% beträgt.

11. Verfahren zum Herstellen einer Abdeckvorrichtung (100) zum Abdecken der Lippen und Mundwinkel eines Patienten während einer Behandlung, bei dem ein Kunststoff mit Duftmolekülen in eine Spritzgussform eingespritzt wird.

12. Speichelsauger (200) zum Absaugen von Speichel aus dem Mund eines Patienten, der einen Kunststoff mit Duftmolekülen umfasst.

13. Speichelsauger (200) nach Anspruch 12, wobei der Speichelsauger (200) ein Röhrchen (203) umfasst, in dem der Kunststoff mit den Duftmolekülen angeordnet ist.

14. Speichelsauger (200) nach Anspruch 13, wobei der Kunststoff mit den Duftmolekülen an der Spitze oder dem Ende des Röhrchens (203) angeordnet ist.

15. Verfahren zum Herstellen eines Speichelsaugers (200), bei dem ein Kunststoff mit Duftmolekülen in eine Spritzgussform eingespritzt wird.
